# EUROPEAN PATENT APPLICATION

(11) **EP 4 509 101 A1**
(43) Date of publication of application: **19.02.2025**
(21) Application number: 23192037.2
(22) Date of filing: 17.08.2023
(51) Int. Cl.: A61F 13/00, A61F 13/01, A61K 9/70, A61L 15/22, A61L 15/26, D01D 5/00, D04H 1/435, D04H 1/4382, D04H 1/728

(54) **A NON-WOVEN ANTI-CHAFING MATERIAL, A METHOD FOR ITS PREPARATION AND AN ANTI-CHAFING PATCH COMPRISING A NON-WOVEN MATERIAL**

(71) Applicant: Markowicz - Jureczko, Urszula, 43-250 Pawlowice (PL)
(72) Inventor: Markowicz - Jureczko, Urszula, 43-250 Pawlowice (PL)
(74) Representative: Tyrala, Magdalena

(57) **Abstract**

A protective material intended to be applied to the skin comprising at least one polymer layer of a non-woven fabric obtained by electrospinning in an electrostatic field is characterized in that the polymer layer comprises a mixture of polycaprolactone (PCL) and polyethylene oxide (PEO) with a predominance of polycaprolactone (PCL) and that the layer of non-woven fabric is spot welded. A protective patch comprising at least one layer of a polymeric non-woven fabric (i), (ii), (iii), an adhesive layer (A) and a removable layer (C) covering the adhesive layer (A), characterized in that the polymer layer (i), (ii), (iii) is a mixture of polycaprolactone (PCL) and polyethylene oxide (PEO), and optionally a mixture of polymers mixed with an excipient, one or more layers together being spot-welded, the adhesive (A), preferably silicone is spot applied at the weld points (B). A method for obtaining a protective material containing at least one polymer non-woven layer obtained by electrospinning in an electrostatic field, characterized in that it comprises the following steps: I. preparation of the initial PCL/PEO solution by mixing polycaprolactone (PCL) and polyethylene oxide (PEO) with predominance of polycaprolactone (PCL) in a mixture of acetone and chloroform with a volume ratio of 70:30, II. transformation of the starting solution obtained in point I. into fibers and non-woven fabric by electro spinning of the starting solution in an electrostatic field, III spot welding of the solution obtained in point II. non-woven fabric at temperatures up to 100°C.

## Description

The subject of the invention is a polymeric non-woven material protecting against skin abrasions. The subject of the invention is also a method of obtaining a nonwoven protective material by electrospinning. The subject of the invention is also a patch protecting against abrasions of the epidermis comprising a polymeric, non-woven protective material obtained by this method.

Many people, especially people with a strong physique or overweight people, struggle with the problem of skin abrasions when moving, for example when walking. Then the inner parts of the thighs rub against each other, causing painful abrasions. Similar abrasions can also occur in other parts of the body, e.g. below the armpits. Therefore, there is a significant need to support them in this problem by providing a product that would eliminate epidermal abrasions.

This problem has so far been solved by wearing tights, longer pants, using gels or using conventional patches. These solutions are inconvenient, especially for people wearing a skirt. Conventional patches, on the other hand, stick strongly to the skin and their removal can be painful.

From the Czech utility model CZ18990U1, a protective patch against abrasions on the inside of the thighs is known. The anti-abrasion patch on the inside of the thighs is made of a transparent material with an adhesive layer, and its shape and size correspond to the overlapping of the friction surfaces created when walking on the inside of the thighs. The adhesive capacity of the adhesive layer is lower than that of conventional medical patchs, so it can be easily removed. The patch can be produced in several sizes and sold in pairs. The protective patch is formed by a cut-out of transparent plastic and is provided on one side with an adhesive layer provided with a protective cover. The protective patch has the shape of a butterfly, i.e. it has the narrowest width s = 10 cm in the middle and at the top and bottom, the end widens to a width of S = 12 cm. The length of the protective patch is 14 cm. The corners of the patch are rounded. The description of the pattern does not reveal what plastic the patch is made of.

The method of electrospinning is known in the art. For example, US2015290354A1 Fig. 1 discloses a typical apparatus for elektroprz 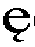 dzenia illustrating the deposition of nanofibers on a collection plate from an electrically charged syringe pump . This electrospinning system basically consists of a metal needle attached to a syringe filled with a polymer solution, a grounded collector, and a high-voltage power supply connected between the needle and the collector, charging them positively and negatively, respectively.

Polymeric non-woven wound protection patches are known in the art. These patches may contain layers containing therapeutic substances, e.g. antibacterial.

The aforementioned publication US2015290354A1 discloses webs comprising fibers obtained by electrospinning comprising a polymer selected from the group consisting of polylactic acid (PLA), polycaprolactone (PCL), polyethylene oxide ( PEO), polyvinyl alcohol (PVA), polyglycolic acid (PGA), poly (ethylene vinyl acetate) (EVA), poly ( ethyleneimine ) (PEI), poly ( 2-hydroxyethyl methacrylate ) ( PHEMA ), methacrylate poly (2-hydroxypropyl) methacrylate poly (2-(dimethylamino)ethyl), polylysine, poly ( methyl methacrylate ) (PMMA), polypyrroles , cyclodextrin , poly (α-[4-aminobutyl]-1-glycolic acid) (PAGA), poly (2-( dimethylamino) ethyl methacrylate ) ( pDMAEMA ), poly ( enol ketone) (PEK ), N-(2-hydroxypropyl) methacrylamide (HPMA) and their blends, derivatives and copolymers. In certain embodiments, the networks may include two or more therapeutic agents. The polymer solution generally comprises an electrospinning polymer or blend of polymers and one or more solvents. The solvent or solvents may be, for example, dichloromethane (DMC), ethylene acetate (EA), dichloroethylene (DCE), dimethylformamide (DMF), hexafluoro isopropanol (HIFP), dichloromethane (DCM), tetrahydrofuran (THF), ethyl acetate (EA), chloroform , acetone, heptane, isopropyl alcohol, octanol and toluene and water. The concentration of the polymer solution may vary, and in some embodiments, the polymer solution may contain about 5% to about 25% by weight of polymer.

US2013150763A1 discloses a wound dressing comprising three polymer layers. The first layer is a load-bearing layer of a hydrophobic nature. The polymers of this layer are selected from the group consisting of polyamides, polycaprolactone (PCL), polylactic acid (PLA), poly (lactic-co-glycolic acid) (PLGA) and combinations thereof. Second polymer layer polymers are selected from the group consisting of polyamides, polycaprolactone (PCL), polylactic acid (PLA), poly (lactic-co-glycolic acid) (PLGA) and combinations thereof, or the hydrophilic polymers are selected from the group consisting of chitosan , gelatin, collagen, polyvinyl alcohol (PVA), polyethylene oxide (PEO), and combinations thereof, wherein the hydrophilic polymer is chitosan . The second nanofibrous layer preferably comprises chitosan and polyethylene oxide (PEO). The second layer also contains Melilotus extract officinalis which is mixed with the second nanofibrous layer in a ratio of 50% or 30% or 10% of the total weight. The third nanofibrous layer contains biocompatible hydrophilic polymers. Biocompatible hydrophilic polymers are selected from the group consisting of chitosan, collagen, gelatin, polyvinyl alcohol (PVA), polyethylene oxide (PEO) and combinations thereof. The third nanofibrous layer consists of chitosan and polyethylene oxide (PEO). The chitosan is present in the third nanofibrous layer in a ratio of 90 weight percent or less of the polymer.

In turn, Taiwanese TWI678019B discloses laying and hot-pressing two layers of polymer fibers, each polymer fiber film has a fiber orientation, and the fiber orientations of the two polymer fiber films are perpendicular to each other; where the hot pressing temperature is 90 °C ~110 °C , the pressure is 10~20kg/cm2, and the duration is 5 minutes.

The object of the invention is to provide a skin-compatible material that could be used for a permanent but easily removable fixation on the skin in areas prone to abrasions.

The protective anti-chafing material according to the invention, intended to be applied to the skin, comprises at least one polymer non-woven layer obtained by electrospinning in an electrostatic field. The material is characterized by the fact that the polymer layer is a mixture of polycaprolactone (PCL) and polyethylene oxide (PEO) with the predominance of polycaprolactone (PCL) and that the non-woven layer is spot welded.

Preferably, the weight ratio of polycaprolactone (PCL) and polyethylene oxide (PEO) in the mixture is 10:1. Preferably, the protective material comprises a nonwoven fabric mixed with an excipient in one or more of the following combinations:
1) polycaprolactone (PCL) / polyethylene oxide (PEO) / aloe vera (AV), with Aloe Vera in the amount of 5% by weight of the total weight of polymers ((polycaprolactone (PCL) + polyethylene oxide (PEO)),
2) polycaprolactone (PCL) / polyethylene oxide (PEO) / Shea butter (MS) / Avocado butter (MA) / Lanolin (L) with Shea butter (MS) in the amount of 2%, Avocado butter (MA) in the amount of 2% and Lanolin (L) in an amount of 1% by weight based on the total weight of the polymers ((polycaprolactone (PCL) + polyethylene oxide (PEO)),
3) poly(vinyl alcohol) (PVA) / Aloe Vera (AV), Aloe Vera (AV) in the amount of 5% by weight in relation to the weight of PVA,
4) polycaprolactone (PCL) / polyethylene oxide (PEO) / pigment (PI) pigment was added in the amount of 5 to 50% by weight, depending on the planned color scheme in relation to the total weight of polymers ((polycaprolactone (PCL) + polyethylene oxide ( PEO)),
5) polycaprolactone (PCL) / polyethylene oxide (PEO) / hyaluronic acid, with hyaluronic acid in the amount of 5% by weight in relation to the total weight of polymers ((polycaprolactone (PCL) + polyethylene oxide (PEO))
6) polycaprolactone (PCL) / polyethylene oxide (PEO) / CBD oil, with CBD oil in the amount of 5% by weight in relation to the total weight of polymers ((polycaprolactone (PCL) + polyethylene oxide (PEO))
7) polycaprolactone (PCL) / polyethylene oxide (PEO) / hemp ointment, with hemp ointment in the amount of 5% by weight in relation to the total weight of polymers ((polycaprolactone (PCL) + polyethylene oxide (PEO))

Preferably, the material comprises three adjacent layers:
(i) an inner layer intended to be applied to the skin composed of polycaprolactone (PCL) and polyethylene oxide (PEO) non-woven fabric, this layer does not contain any additives,
(ii) a middle layer, which is a carrier of active substances, made of a non-woven fabric, the composition of which includes fibers obtained from combination 1). polymers of polycaprolactone (PCL), polyethylene oxide (PEO) and an active substance such as aloe vera (AV), 2). polymers of polycaprolactone (PCL), polyethylene oxide (PEO) and active substances such as shea butter (MS), avocado butter (MA), lanolin (L), 3). poly(vinyl alcohol) (PVA) and aloe vera (AV), and
(iii) an outer layer being pigment carrier layer comprising a non-woven fabric made of a combination of the polymers: polycaprolactone (PCL), polyethylene oxide (PEO) and pigment (PI).

In another variant, the material preferably comprises three adjacent layers:
(i) an inner layer - intended to be applied to the skin made of non-woven fabric, the composition of which includes fibers obtained from a combination of 1) polycaprolactone (PCL) / polyethylene oxide (PEO) polymers and an active substance such as aloe vera (AV), 2. polycaprolactone (PCL) polymers, polyethylene oxide (PEO) and active substances such as shea butter (MS), avocado butter (MA), lanolin (L), poly(vinyl alcohol) (PVA) and aloe vera (AV),środkow 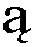 - b 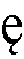 d 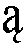 c 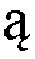 nośnikiem substancji aktywnych, zbudowan 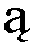 z w 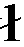 ókniny polikaprolaktonu (PCL) i politlenku etylenu (PEO),
(ii) a middle layer - a carrier of active substances, made of polycaprolactone (PCL) and polyethylene oxide (PEO) non-woven fabric,
(iii) an outer layer, which is a pigment carrier, contains a non-woven fabric made of a combination of polymers: polycaprolactone (PCL), polyethylene oxide (PEO) and pigment (PI).

The essence of the invention is also a protective, anti-chafing patch comprising at least one layer of a polymer non-woven fabric, an adhesive layer and a removable layer constituting a cover for the adhesive layer. The patch is characterized in that the polymer layer is a mixture of polycaprolactone (PCL) and polyethylene oxide (PEO), and optionally a mixture of polymers mixed with an excipient in one or more of the following combinations:
1) polycaprolactone (PCL) / polyethylene oxide (PEO) / aloe vera (AV), with Aloe Vera in the amount of 5% by weight of the total weight of polymers ((polycaprolactone (PCL) + polyethylene oxide (PEO))
2) polycaprolactone (PCL) / polyethylene oxide (PEO) / Shea butter (MS) / Avocado butter (MA) / Lanolin (L) with Shea butter (MS) in the amount of 2%, Avocado butter (MA) in the amount of 2% and Lanolin (L) in an amount of 1% by weight based on the total weight of the polymers ((polycaprolactone (PCL) + polyethylene oxide (PEO)),
3) poly(vinyl alcohol) (PVA) / Aloe Vera (AV), Aloe Vera (AV) in the amount of 5% by weight in relation to the weight of PVA
4) polycaprolactone (PCL) / polyethylene oxide (PEO) / pigment (PI) pigment was added in the amount of 5 to 50% by weight, depending on the planned color scheme in relation to the total weight of polymers ((polycaprolactone (PCL) + polyethylene oxide (PEO))
5) polycaprolactone (PCL) / polyethylene oxide (PEO) / hyaluronic acid, with hyaluronic acid in the amount of 5% by weight in relation to the total weight of polymers ((polycaprolactone (PCL) + polyethylene oxide (PEO))
6) polycaprolactone (PCL) / polyethylene oxide (PEO) / CBD oil, with CBD oil in the amount of 5% by weight in relation to the total weight of polymers ((polycaprolactone (PCL) + polyethylene oxide (PEO))
7) polycaprolactone (PCL) / polyethylene oxide (PEO) / hemp ointment, with hemp ointment in the amount of 5% by weight in relation to the total weight of polymers ((polycaprolactone (PCL) + polyethylene oxide (PEO))
wherein the one or more layers together are spot welded, wherein the silicone adhesive is spot applied at the weld points.

In a preferred variant of the patch, the weight ratio of polycaprolactone (PCL) and polyethylene oxide (PEO) is 10:1.

Preferably, the patch comprises one of the following combinations of layers:
a) three layers and silicone glue applied to the inner layer:
   (i) - inner layer - in direct contact with the body, polymer nonwovens of PCL and/or PEO/AV and/or PCL/PEO/MS/MA/L and/or PVA/AV were used for this purpose.
   (ii) - middle layer - a carrier of active substances, for this purpose PCL/PEO polymer nonwovens were used
   (iii) - outer layer - a carrier of pigment and a combination of PCL/PEO/PI fibres, or
b) two layers and silicone glue applied to the inner layer:
   (i) inner layer - in direct contact with the body, for this purpose, non-woven fabrics were used that are a combination of PCL/PEO/AV and/or PCL/PEO/MS/MA/L and/or PVA/AV and/or and PCL/PEO. On the outer part of the layer, silicone was applied pointwise for sticking the patch to the body
   (ii) outer layer - a carrier of pigment and a combination of PCL/PEO/PI fibres
      a) one layer with silicone adhesive applied to it, comprising one layer (i) in direct contact with the body of non-woven fabrics made of a combination of the following fibers: PCL/PEO/AV and/or PCL/PEO/MS/MA/L and/or PVA/AV and/or PCL/PEO and PCL/ PEO/PI.

The essence of the invention is also a method of obtaining a protective, anti-chafing material containing at least one polymer layer of non-woven fabric obtained by electrospinning in an electrostatic field, which is characterized in that it comprises the following steps:
I. preparation of the starting PCL/PEO solution by mixing polycaprolactone (PCL) and polyethylene oxide (PEO) with a predominance of polycaprolactone (PCL) in a mixture of acetone and chloroform with a volume ratio of 70:30 until complete dissolution,
II. converting the starting solution obtained in point I into fibers and non-woven fabrics by electrospinning the starting solution in an electrostatic field.
III. spot welding obtained in point II. non-woven fabric at temperatures up to 100°C.

In a preferred variant of the process, step I additionally comprises the preparation of one or more starting solutions:
1) PCL/PEO/AV by mixing Aloe Vera powder in a mixture of acetone and chloroform with a volume ratio of 70:30 under ultrasound for 1 minute, then adding polycaprolactone (PCL) and polyethylene oxide (PEO) and mixing until completely dissolution of polymeric materials,
2) PCL/PEO/MS/MA/L by mixing Shea Butter, Avocado Butter and Lanolin in a mixture of acetone and chloroform with a volume ratio of 70:30 in a total amount of up to 5% by weight in relation to the total weight of polymers, and then adding polycaprolactone (PCL) and polyethylene oxide (PEO) and mixing until complete dissolution of polymeric materials,
3) PVA/AV by mixing distilled water with poly(vinyl alcohol) (PVA) granulate under the influence of a temperature of about 90 °C until the granulate is completely dissolved, and then cooling the solution to a temperature of about 20 to 25 °C, and then adding Aloe Vera powder to the solution in the amount of 5% and subjected to dissolution for a period of about 12 hours,
4) PCL/PEO/PI by mixing the pigment in a mixture of acetone and chloroform with a volume ratio of 70:30 under ultrasound for 1 minute, and then adding polycaprolactone (PCL) and polyethylene oxide (PEO) and stirring until the materials are completely dissolved polymers, with the pigment added in the amount of 5 to 50% by weight, depending on the color scheme planned to be obtained in relation to the total weight of the polymers.
5) polycaprolactone (PCL) / polyethylene oxide (PEO) / hyaluronic acid, with hyaluronic acid in the amount of 5% by weight in relation to the total weight of polymers ((polycaprolactone (PCL) + polyethylene oxide (PEO))
6) polycaprolactone (PCL) / polyethylene oxide (PEO) / CBD oil, with CBD oil in the amount of 5% by weight in relation to the total weight of polymers ((polycaprolactone (PCL) + polyethylene oxide (PEO))
7) polycaprolactone (PCL) / polyethylene oxide (PEO) / hemp ointment, with hemp ointment in the amount of 5% by weight in relation to the total weight of polymers ((polycaprolactone (PCL) + polyethylene oxide (PEO))
step II comprises: transformation of the initial PCL/PEO solution and one or more initial solutions 1) - 7) obtained in point I. into fibers and non-woven fabric, by electro-spinning of the initial solutions in an electrostatic field using separate nozzles for each initial solution.

In a preferred variant of the method, the three-layer protective material is obtained by electrospinning the starting PCL/PEO solution and obtaining the first inner layer (i), then electrospinning one or more starting solutions 1) to 7) directly onto the first layer (i) and obtaining the second layer - the middle one (ii) which is a carrier of active substances, then electrospinning the starting solution 5) directly onto the second layer (ii) to obtain the third outer layer (iii).

In a preferred variant of the method, the three-layer protective material is obtained by electrospinning one or more starting solutions 1) to 7) directly and obtaining the first - inner layer (i) and then electrospinning the starting PCL/PEO solution directly onto the first layer and obtaining the second - middle layer ( ii), then electrospinning the starting solution 4) directly onto the second layer (ii) to obtain the third outer layer (iii).

In another preferred variant of the method, a two-layer protective material is obtained by electrospinning one or more of the starting solutions 1) to 7) directly and obtaining the first - inner layer and then the starting solution 4) directly on the second layer to obtain the second - outer layer (ii).

In another preferred variant of the method, a single-layer protective material is obtained by simultaneously electrospinning multiple starting solutions 1) to 7) and obtaining the first layer (i).

Thanks to the invention, a material that is very pleasant to the touch has been obtained, which at the same time has good strength.

Various aspects of the invention are shown in the drawing in which:
Figure 1 shows an exemplary patch in the shape of a rectangle,
Figure 2 is a cross-sectional view of a patch comprising one layer of a polymeric material dotted with silicone
Figure 3 is a cross-sectional view of a patch comprising two layers of polymeric material dotted with silicone
Figure 4 is a cross-sectional view of a patch comprising three layers of polymeric material dotted with silicone.

In the following description, the individual substances have the following definitions:
Shea butter - vegetable oil obtained from the fruit of Parka (called shea tree). In its fresh, unprocessed form, it has a paste-like consistency, is whitish in color, is almost odorless and has a very faint taste.

Avocado butter produced in the process of hydrogenation of cold-pressed avocado oil.

Aloe vera powder is the hardened juice of the leaves of various species of aloe vera. The raw material should contain not less than 18% of anthranoid compounds, expressed as aloin. It has the form of lumps of irregular shape and various sizes, green-brown, almost black in color, usually covered with green dust; the lumps disintegrate into sharp-edged fragments on compression; their fracture is shiny, shell-like, a peculiar smell.

Lanolin, otherwise fat sweat - animal wax obtained when cleaning sheep's wool. It is a mixture of fatty acid esters with sterols (including cholesterol). Pure, anhydrous lanolin is in the form of a thick, dark yellow or yellow-brown mass with a faint, slightly alcoholic smell. Hyaluronic acid - an organic chemical compound, a polysaccharide from the group of glycosaminoglycans. Hyaluronic acid is a biopolymer with alternating D-glucuronic acid and N-acetyl-D-glucosamine units connected by β (1→4) and β (1→3) glycosidic bonds.

CBD hemp oil - is an extract obtained from hemp seeds and flowers in the form of oil, containing cannabidiol in short called CBD. It has a green-brown color and a slightly bitter, spicy-nut aroma and taste.

Hemp ointment - skin ointment composed of the following ingredients: Aqua, Cannabis Sativa (Hemp) Seed Oil, Alcohol Denat., Propylene Glycol, Glycerin, Copernicia Cerifera (Carnauba Wax) Cera, Cetearyl Alcohol, Glyceryl Stearate, Petrolatum, Cetearyl Glucoside, Phenoxyethanol , Ethylhexylglycerin, PEG-40 Hydrogenated Castor Oil, Camphor, Methyl Salicylate, Turpentine, Xanthan Gum, Menthol, Carbomer, Tocopherol, D-Limonene (the content of ingredients is reserved by the manufacturer).

In the following, the following abbreviations will be used for brevity and clarity of description: PCL for polycaprolactone, PEO for polyethylene oxide, AV for Aloe Vera. MS for Shea Butter, MA for Avocado Butter, L for Lanolin, PVA for polyvinyl alcohol and PI for pigment.

### Example 1

An example of a protective material is a non-woven material made of the following polymers: a combination of polymers of polycaprolactone (PCL) and polyethylene oxide (PEO) and poly(vinyl alcohol) (PVA) and the substances: aloe vera, shea butter, avocado butter and lanolin. The material consists of three adjacent and adjacent layers:
(i) the inner layer, intended to be in direct contact with the body surface and made of non-woven polycaprolactone (PCL) and polyethylene oxide (PEO), this layer does not contain any additives,
(ii) the middle leyer, which is a carrier of active substances, made of a non-woven fabric, the composition of which includes fibers obtained from a combination of a) polymers of polycaprolactone (PCL), polyethylene oxide (PEO) and an active substance such as aloe vera (AV), b) polymers of polycaprolactone (PCL) , polyethylene oxide (PEO) and active substances such as shea butter (MS), avocado butter (MA), lanolin (L), c) poly(vinyl alcohol) (PVA) and aloe vera (AV), d) poly(vinyl alcohol) vinyl) (PVA) and aloe vera microcapsules (KAV) and
(iii) an outer layer, which is pigment carrier layer comprising a non-woven fabric made of a combination of the polymers: polycaprolactone (PCL), polyethylene oxide (PEO) and pigment (PI).

The pigments are titanium dioxide and iron oxide combined. As a result of their combination, a color resembling the color of flesh is created. Titanium dioxide is in the form of microparticles.

The non-woven material is obtained in the electrospinning process from solutions of polymers dissolved in:
a) a combination of: acetone and chloroform in the ratio: 70:30, for polycaprolactone (PCL) / polyethylene oxide (PEO), and
b) distilled water for Poly(vinyl alcohol) (PVA).

The layers: inner (i), middle (ii) and outer (iii) are applied to each other and the three-layer material is spot welded. Spot welding is carried out, for example, with a splined roller heated to 100 °C. Spot welding increases the strength of the multi-layer non-woven fabric.

The method of obtaining the material according to the invention.

The protective material according to the invention was obtained in three sub-steps (I, II, III) which are described below.

### Sub-stage I - preparation of solutions

To obtain the protective material, initial solutions for individual fiber combinations were prepared first.

### 1) PCL/PEO fibers

Acetone and chloroform were placed in the tank with a cap in a volume ratio of 70:30, and then the polymers: polycaprolactone (PCL), polyethylene oxide (PEO) were added in a weight ratio of PCL:PEO equal to 10:1 and mixed until the polymeric materials were completely dissolved (PCL, PEO) and thus the creation of a PCL/PEO solution.

A much higher content of PCL in relation to PEO results in a very velvety material in contact. If the ratio was reversed, the resulting fibers would dissolve on contact with water, as polyethylene oxide (PEO) is sensitive to water. On the other hand, a higher content of PCL gives a higher strength of the material.

### 2) PCL/PEO/AV fibers

Acetone and chloroform were placed in the tank with a cap in a 70:30 volume ratio, then Aloe Vera powder was added in an amount of 5% by weight in relation to the total weight of polymers ((polycaprolactone (PCL) + polyethylene oxide (PEO)) and subjected to intensive mixing and simultaneous ultrasonics for 1 minute using a sonotrode, after the set time the sonotrode was removed, polymers polycaprolactone (PCL), polyethylene oxide (PEO) were added in a weight ratio of 1:10 and mixed until the polymeric materials were completely dissolved and thus a polycaprolactone solution was formed ( PCL)/ polyethylene oxide (PEO)/ aloe vera (AV).

### 3) PCL/PEO/MS/MA/L fibers

Acetone and chloroform were placed in a tank with a cap in a volume ratio of 70:30, then Shea Butter (MS) in the amount of 2%, Avocado Butter (MA) in the amount of 2% and Lanolin (L) in the amount of 1% were added, the total weight of the additives used did not exceed 5% by weight in relation to the total weight of polymers ((polycaprolactone (PCL) + polyethylene oxide (PEO)), the mentioned additives were intensively mixed for 30 minutes, after the set time polymers polycaprolactone (PCL), polyethylene oxide (PEO) were added ) in a weight ratio of 1:10 and were mixed until complete dissolution of polymeric materials (PCL, PEO) and thus a solution of polycaprolactone (PCL)/polyethylene oxide (PEO)/ Shea butter (MS)/ Avocado butter (MA)/ Lanolin (L)

### 4) PVA/AV fibers

Distilled water and poly(vinyl alcohol) (PVA) granules were placed in a container with a cap, and the combined materials were intensively mixed and subjected to a temperature of 90□C for 2 hours. The effect of temperature and time enabled the poly(vinyl alcohol) (PVA) granulate to be completely dissolved, after dissolution, the obtained solution was cooled until the temperature reached 20-25□C, Aloe Vera (AV) powder was added to the cooled solution in the amount of 5% by weight in relation to to the mass of PVA and dissolved for 12 h, which finally led to the formation of a solution of poly(vinyl alcohol) (PVA) / Aloe Vera (AV)

### 5) PCL/PEO/PI fibers

Acetone and chloroform were placed in the tank with a cap in a ratio of 70:30, then the pigment was added in an amount of 5 to 50% by weight, depending on the planned color scheme in relation to the total weight of polymers ((polycaprolactone (PCL) + polyethylene oxide (PEO) ) and subjected to intensive mixing and simultaneous ultrasound for 1 minute using a sonotrode, after the set time the sonotrode was removed, polymers polycaprolactone (PCL) + polyethylene oxide (PEO) were added in a weight ratio of 1:10 and stirred until complete dissolution of polymeric materials (PCL, PEO) and thus a solution of polycaprolactone (PCL) / polyethylene oxide (PEO) / pigment (PI).

### Sub-stage II - transformation of solutions into fibers and then nonwovens

To obtain the target protective material, three directly applied layers were obtained:
(i) internal - in direct contact with the body, a solution of polycaprolactone (PCL) / polyethylene oxide (PEO) was used for this purpose, which was transformed with the use of an electrostatic field into fibers and then into a non-woven fabric,
(ii) the middle one - a carrier of active substances, solutions of poly(vinyl alcohol) (PVA) / Aloe Vera (AV), polycaprolactone (PCL) / polyethylene oxide (PEO) / Shea butter (MS) / Avocado butter ( MA)/ Lanolin (L), poly(vinyl alcohol) (PVA)/Aloe Vera (AV) solution, which was transformed by electrostatic field into fibers and then into a non-woven fabric,
(iii) outer - pigment carrier polycaprolactone (PCL)/polyethylene oxide (PEO)/pigment (PI), which was transformed using an electrostatic field into fibers and then into a non-woven fabric.

An electrostatic field of 1 to 1.5 kV/cm was used for all fibers.

According to the invention, the conversion of the solutions into nonwoven fabrics proceeded as follows:
a) for the inner layer (i): PCL/PEO fibers were applied directly to the surface of the collector, which, falling onto the collector, formed a non-woven fabric. nozzles or nozzles) and subjected to the action of an electrostatic field, with the use of which, at the distance between the electrodes, the solvents used were evaporated, leading to the formation of fibers falling on the surface of the collector, forming the structure of the non-woven fabric being the inner layer, then
b) for the middle layer (ii): using three independent nozzles separately for PCL/PEO/AV, PCL/PEO/MS/MA/L, PVA/AV, PCL/PEO fibers were applied directly to the surface of the previously obtained PCL/PEO nonwoven /AV, PCL/PEO/MS/MA/L, PVA/AV, PVA/KAV for this purpose PCL/PEO/AV, PCL/PEO/MS/MA/L, PVA/AV solutions, using infusion pumps, tanks for the solution and polytetrafluoroethylene (PTFE) tubes) were transported to the positive electrodes (nozzle) and subjected to the action of an electrostatic field with the use of which the applied solvents evaporated at the distance between the electrodes, leading to the formation of fibers 1) PCL/PEO/AV, 2) PCL/ PEO/MS/MA/L, 3) PVA/AV, falling onto the surface of the collector forming the structure of the non-woven fabric being the middle layer, then
c) for the outer layer (iii): PCL/PEO/PI fibers were applied to the surface of the two previously created layers, which, falling onto the collector, formed a non-woven fabric for this purpose, PCL/PEO/PI solution using infusion pumps, solution tanks and Poly(tetrafluoroethylene) tubes (PTFE) is transported to the positive electrode (nozzles or nozzles) and subjected to an electrostatic field with the use of which, at the distance between the electrodes, the solvents used were evaporated, leading to the formation of fibers falling on the surface of the collector, forming the structure of the non-woven fabric being the outer layer.

### Sub-stage III - welding layers

According to the invention, the resulting layered nonwoven protective material structure with an inner layer (i), a middle layer (ii) and an outer layer (iii) is then subjected to spot welding. This action is possible thanks to the use of thermoplastic (PCL), which dominates the protective material.

The layers are spot welded using a studded roller heated to 100 °C. As a result of spot welding, the strength of the multilayer non-woven fabric is increased.

The protective material may have, for example, a thickness of 0.2 mm to 3 mm. The preferred thickness is 2 mm.

The protective material can also be applied to other media, materials, and fabrics. A person skilled in the art will find many possibilities to combine the protective material with other materials as desired.

To prepare the protective patch material, hypoallergenic silicone adhesive A, adapted to the size of the welded surface, is applied at the welding points B on the inner layer (i). protective patch on the surface of the body. Protective layers for patches are known in the art and a skilled person is able to use a protective layer suitable for silicone adhesive.

### Example 2

In another example, the protective material consists of three adjacent and directly applied layers:
(i) an inner layer - intended for direct contact with the skin and being a carrier of bioactive substances, made of non-woven fabric, the composition of which includes fibers obtained from a combination of a) polycaprolactone (PCL) / polyethylene oxide (PEO) polymers and an active substance such as aloe vera ( AV), b) polymers of polycaprolactone (PCL), polyethylene oxide (PEO) and active substances such as shea butter (MS), avocado butter (MA), lanolin (L), c) poly(vinyl alcohol) (PVA) and aloe vera (AV),
(ii) a middle layer - made of non-woven polycaprolactone (PCL) and polyethylene oxide (PEO),
(iii) an outer layer - which is a pigment carrier, containing a non-woven fabric made of a combination of polymers: polycaprolactone (PCL), polyethylene oxide (PEO) and pigment (PI).

The pigments are titanium dioxide and iron oxide combined. As a result of their combination, a color resembling the color of flesh is created. Titanium dioxide is in the form of microparticles.

The protective material was created by electrospinning from solutions of polymers dissolved in:
1) a combination of: acetone and chloroform in the volume ratio: 70:30, for polycaprolactone (PCL) / polyethylene oxide (PEO) and
2) distilled water for Poly(vinyl alcohol) (PVA).

The layers: inner (i), middle (ii) and outer (iii) are applied to each other and the three-layer material is spot welded. Spot welding is carried out, for example, with a splined roller heated to 100 °C. Spot welding increases the strength of the multi-layer non-woven fabric.

The protective material was obtained as described below.

### Sub-stage I - preparation of solutions

The solutions were prepared as in Example 1.

### Sub-stage II - transformation of solutions into fibers and then nonwovens

To obtain the target protective material described above, three directly applied layers (i), (ii), (iii) were obtained.

According to the invention, the transformation of the solutions into nonwoven fabrics proceeded as follows:
(a) for the inner layer (i): fibers were applied directly to the surface of the collector using three independent nozzles, separate for PCL/PEO/AV, PCL/PEO/MS/MA/L, PVA/AV solutions, directly on the surface previously obtained non-woven fabric 1) PCL/PEO fibers were applied 2) PCL/PEO/AV, 3) PCL/PEO/MS/MA/L, 4) PVA/AV, for this purpose PCL/PEO/AV, PCL/PEO/ MS/MA/L, PVA/AV, using infusion pumps, solution tanks and poly(tetrafluoroethylene) (PTFE) tubes, were transported to the positive electrodes (nozzle) and subjected to an electrostatic field, where the solvents used were evaporated at the distance between the electrodes leading to the formation of fibers 2) PCL/PEO/AV, 3) PCL/PEO/MS/MA/L, 4) PVA/AV, falling onto the surface of the collector forming the structure of the non-woven fabric being the inner layer, then
(b) for the middle layer (ii): on the layer (i) thus formed, 1) PCL/PEO fibers are applied, which, falling onto the collector, formed a non-woven fabric for this purpose, a PCL/PEO solution using infusion pumps, solution tanks and Poly( tetrafluoroethylene) (PTFE) is transported to the positive electrode (nozzles or nozzles) and subjected to the action of an electrostatic field with the use of which the solvents used at the distance between the electrodes were evaporated, leading to the formation of fibers falling on the surface of the collector forming the structure of the non-woven fabric being the middle layer, then
(c) for the outer layer (iii): on the surface of the two previously created layers (i, ii) 6) PCL/PEO/PI fibers were applied, which, falling on the collector, formed a non-woven fabric, for this purpose PCL/PEO/PI solution using infusion pumps, tanks for the solution and poly(tetrafluoroethylene) PTFE tubes is transported to the positive electrode (nozzles or nozzles) and subjected to the action of an electrostatic field, with the use of which, at the distance between the electrodes, the solvents used were evaporated, leading to the formation of fibers falling on the surface of the collector, forming the structure of the non-woven fabric which is the outer layer.

### Sub-stage III - welding layers

According to the invention, the resulting nonwoven structure of the protective material is then subjected to spot welding. This action is possible thanks to the use of thermoplastic (PCL), which dominates the protective material. The layers are spot welded using a studded roller heated to 100oC. As a result of spot welding, the strength of the multilayer non-woven fabric is increased.

The protective material may have, for example, a thickness of 0.2 mm to 3 mm. The preferred thickness is 2 mm.

The protective material can also be applied to other media, materials and fabrics. A person skilled in the art will find many possibilities to combine the protective material with other materials as desired.

To prepare the protective patch material, hypoallergenic silicone adhesive A, adapted to the size of the welded surface, is applied at the welding points B on the inner layer (i). protective patch on the surface of the body. Protective layers for patches are known in the art and a skilled person can use a protective layer suitable for silicone adhesive.

### Example 3

In another example, the protective material comprises two directly applied layers:
(i) the internal layer, in direct contact with the skin, which is a combination of polymer nonwovens PCL/PEO/AV, PCL/PEO/MS/MA/L, PVA/AV, PCL/PEO
(ii) the outer layer, which is a pigment carrier and is a combination of PCL/PEO/PI fibres.

The protective material was created by electrospinning from solutions of polymers dissolved in:
a) a combination of: acetone and chloroform in the volume ratio: 70:30, for polycaprolactone (PCL) / polyethylene oxide (PEO), and
b) distilled water for Poly(vinyl alcohol) (PVA).

The method of obtaining the material according to the invention.

### Sub-stage I - preparation of solutions

To obtain the protective material, the starting solutions for the fibers mentioned in examples 1 and 2 were first prepared in the manner described in these examples.

### Sub-stage II - transformation of solutions into fibers and then nonwovens

To obtain the target protective material, three directly applied layers were obtained:
(i) an internal layer - in direct contact with the body, solutions of 2) PCL/PEO/AV, 3) PCL/PEO/MS/MA/L, 4) PVA/AV, 1) PCL/PEO were used for this purpose, which were transformed with electrostatic field into the fibers and then the non-woven fabric
(ii) an outer layer - a pigment carrier, a PCL/PEO/PI solution was used for this purpose, which was transformed with the use of an electrostatic field into fibers and then a non-woven fabric

According to the invention, the transformation of the solutions into nonwoven fabrics proceeded as follows:
a) for the inner layer (i): fibers were applied directly to the collector surface using four independent nozzles, separately for PCL/PEO/AV, PCL/PEO/MS/MA/L, PVA/AV, PCL/PEO solutions, with the use of infusion pumps, tanks for the solution and PTFE tubes were transported to the positive electrodes (nozzles) and subjected to the action of an electrostatic field with the use of which the applied solvents evaporated at the distance between the electrodes, leading to the formation of fibers 2) PCL/PEO/AV, 3) PCL/ PEO/MS/MA/L, 4) PVA/AV, 1) PCL/PEO falling on the surface of the collector forming the structure of the non-woven fabric being the inner layer, then
b) for the outer layer (ii): on the surface of the previously created layer (i) PCL/PEO/PI fibers were applied, which falling onto the collector formed a non-woven fabric for this purpose PCL/PEO/PI solution using infusion pumps, solution tanks and PTFE tubes is transported to the positive electrode (nozzles or nozzles) and subjected to the action of an electrostatic field with the use of which, at the distance between the electrodes, the solvents used were evaporated, leading to the formation of fibers falling on the surface of the collector, forming the structure of the non-woven fabric which is the outer layer.

### Podetap III - zgrzewanie warstw

### Sub-stage III - welding layers

According to the invention, the resulting nonwoven (protective material) structure is then subjected to spot welding, as in the above-described examples 1 and 2.

The resulting protective material may have exemplary thicknesses of 0.2 mm to 3 mm. The preferred thickness is 2 mm.

The protective material can also be applied to other media, materials and fabrics. A person skilled in the art will find many possibilities to combine the protective material with other materials as desired.

To obtain a patch, an adhesive and a protective material are applied to the material as described in Examples 1 and 2.

### Example 4

In the next example, the protective material contains one layer that is a combination of:
(i) combinations of PCL/PEO/AV, PCL/PEO/MS/MA/L, PVA/AV, PCL/PEO and PCL/PEO/PI polymer nonwovens.

The protective material was created by electrospinning from solutions of polymers dissolved in:
a) a combination of: acetone and chloroform in the volume ratio: 70:30, for polycaprolactone (PCL) / polyethylene oxide (PEO), and
b) distilled water for Poly(vinyl alcohol) (PVA).

In sub-stage I - preparation of solutions, the solutions were prepared in the same way as described in example 1.

### Sub-stage II - transformation of solutions into fibers and then nonwovens

To obtain the target protective material, fibers from the following solutions were applied at the same time: PCL/PEO/AV, PCL/PEO/MS/MA/L, PVA/AV, PVA/KAV, PCL/PEO and PCL/PEO/PI, which converted using an electrostatic field into fibers and then into a non-woven fabric.

According to the invention, the transformation of the solutions into nonwoven fabrics proceeded as follows:
a) for layer (i): fibers were applied directly to the collector surface using five independent nozzles separately for solutions of 2) PCL/PEO/AV, 3) PCL/PEO/MS/MA/L, 4) PVA/AV, 1) PCL/PEO and 5) PCL/PEO/PI directly onto the surface of the collector, for this purpose solutions of 2) PCL/PEO/AV, 3) PCL/PEO/MS/MA/L, 4) PVA/AV, 1) PCL /PEO and 5) PCL/PEO/PI with the use of infusion pumps, tanks for the solution and PTFE tubes were transported to the positive electrodes (nozzle) and subjected to an electrostatic field with the use of which solvents were evaporated at the distance between the electrodes, leading to the formation of PCL fibers/ PEO/AV, PCL/PEO/MS/MA/L, PVA/AV, PCL/PEO and PCL/PEO/PI) falling onto the surface of the collector forming a non-woven structure.

### Sub-stage III - welding

According to the invention, the resulting nonwoven structure of the protective material is then subjected to spot welding, as described in examples 1 and 2.

The protective material may have, for example, a thickness of 0.2 mm to 3 mm. The preferred thickness is 2 mm.

The protective material can also be applied to other media, materials and fabrics. A person skilled in the art will find many possibilities to combine the protective material with other materials as desired.

In the examples described above, hyaluronic acid, CBD oil, hemp ointment were also used as additives. Analogically to the solutions 2) - 4) of Example 1, their content is up to 5% by weight based on the total weight of the polymers ((polycaprolactone (PCL) + polyethylene oxide (PEO)).

To obtain a patch, an adhesive and a protective material are applied to the material as described in Examples 1 and 2.

### Example 5 - protective patch

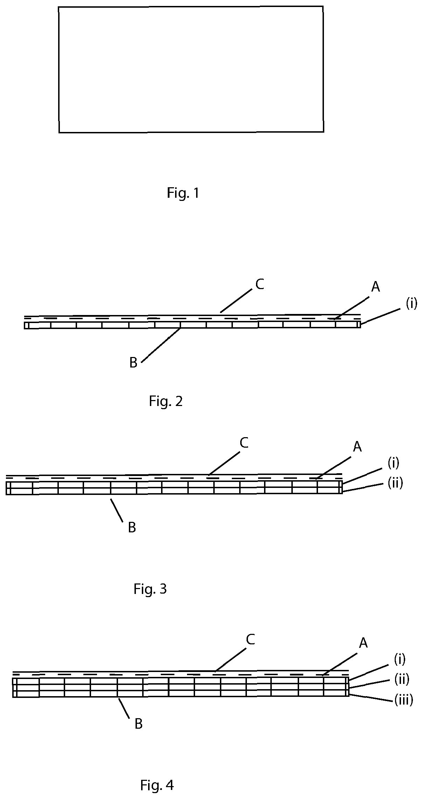

An example of an anti-abrasion patch (Fig. 1 - 4) is a fragment of the protective material described in examples 1 to 4, on the layer intended for contact with the body surface (i), there is a hypoallergenic silicone adhesive applied dots at the welding points B A. The material with the applied adhesive layer A is covered with a removable protective layer C, preventing the material from sticking together. The patch is applied to the skin from the side of the adhesive layer after tearing off the protective layer C.

The patch can be present in three exemplary layers:
a) three layers and silicone glue applied on the inner layer (Fig. 2) and a removable protective layer:
   (i) the internal layer, in direct contact with the body, polymer nonwovens of PCL and/or PEO/AV and/or, PCL/PEO/MS/MA/L and/or PVA/AV were used for this purpose. On the outer part of the layer, silicone was applied pointwise for sticking the patch to the body
   (ii) the middle layer - which is a carrier of active substances, a PCL/PEO solution was used for this purpose
   (iii) the outer layer, which is a pigment carrier and is a combination of PCL/PEO/PI fibres
b) two layers and silicone glue applied on the inner layer (Fig.3) and a removable protective layer:
   (i) - internal - in direct contact with the body, PCL/PEO/AV and/or PCL/PEO/MS/MA/L and/or PVA/AV and PCL/PEO nonwovens were used for this purpose. On the outer part of the layer, silicone was applied pointwise for sticking the patch to the body
   (ii) the outer layer, which is a pigment carrier and is a combination of PCL/PEO/PI fibres
c) one layer (i), in direct contact with the body PCL/PEO/AV and/or PCL/PEO/MS/MA/L and/or PVA/AV and/or PCL/PEO and PCL/PEO/PI nonwovens were used for this purpose. and silicone adhesive applied to it (Fig. 4) and a removable protective layer.

The patch may be in the shape of a rectangle measuring 80 mm x 100 mm. The patch can also have any other shape that is comfortable to wear. Preferably, the thickness of the protective material used in the patch is 0.2 mm to 3 mm, more preferably 2 mm. Depending on the needs, this thickness can also be outside the ranges.

The invention finds application in the cosmetic and medical industries.

## Claims

1. A protective material, intended to be applied to the skin, comprising at least one polymer layer of a non-woven fabric obtained by electrospinning in an electrostatic field, **characterized in that** the polymer layer comprises a mixture of polycaprolactone (PCL) and polyethylene oxide (PEO) with a predominance of polycaprolactone (PCL) and that the nonwoven layer is spot welded.

2. The material according to claim 1, **characterized in that** the weight ratio in the mixture of polycaprolactone (PCL) and polyethylene oxide (PEO) is 10:1.

3. A fabric according to claim 1 or 2, **characterized in that** it comprises a non-woven fabric mixed with an excipient in one or more of the following combinations:
1) polycaprolactone (PCL) / polyethylene oxide (PEO) / aloe vera (AV), with Aloe Vera in the amount of 5% by weight in relation to the total weight of polymers polycaprolactone (PCL) and polyethylene oxide (PEO),
2) polycaprolactone (PCL) / polyethylene oxide (PEO) / Shea butter (MS) / Avocado butter (MA) / Lanolin (L) with Shea butter (MS) in the amount of 2%, Avocado butter (MA) in the amount of 2% and Lanolin (L) in an amount of 1% by weight based on the total weight of the polymers polycaprolactone (PCL) and polyethylene oxide (PEO),
3) poly(vinyl alcohol) (PVA) / Aloe Vera (AV), Aloe Vera (AV) in the amount of 5% by weight in relation to the weight of PVA,
4) polycaprolactone (PCL) / polyethylene oxide (PEO) / pigment (PI) the pigment was added in an amount of 5 to 50% by weight, depending on the planned color scheme in relation to the total weight of polymers polycaprolactone (PCL) and polyethylene oxide (PEO),
5) polycaprolactone (PCL) / polyethylene oxide (PEO) / hyaluronic acid, with hyaluronic acid in the amount of 5% by weight in relation to the total weight of polymers polycaprolactone (PCL) and polyethylene oxide (PEO),
6) polycaprolactone (PCL) / polyethylene oxide (PEO) / CBD oil, with CBD oil in the amount of 5% by weight in relation to the total weight of polymers polycaprolactone (PCL) and polyethylene oxide (PEO),
7) polycaprolactone (PCL) / polyethylene oxide (PEO) / hemp ointment, with hemp ointment in the amount of 5% by weight in relation to the total weight of polymers polycaprolactone (PCL) and polyethylene oxide (PEO),

4. The material according to claim 1 or 2 or 3, **characterized in that** it comprises three adjacent layers:
(i) the inner layer, intended to be applied to the skin, made of non-woven fabric of polycaprolactone (PCL) and polyethylene oxide (PEO), this layer does not contain any additives,
(ii) the middle layer, which is a carrier of active substances, made of a non-woven fabric, the composition of which includes fibers obtained from combination:
1). polymers of polycaprolactone (PCL), polyethylene oxide (PEO) and an active substance such as aloe vera (AV),
2). polymers of polycaprolactone (PCL), polyethylene oxide (PEO) and active substances such as shea butter (MS), avocado butter (MA), lanolin (L),
3). poly(vinyl alcohol) (PVA) and aloe vera (AV),
(iii) a pigment-carrying outer layer comprising a non-woven fabric made of a combination of the polymers: polycaprolactone (PCL), polyethylene oxide (PEO) and pigment (PI).

5. The material according to claim 1 or 2 or 3 **characterized in that** it comprises three adjacent and adjacent layers:
(i) internal layer, intended to be applied to the skin, made of non-woven fabric, the composition of which includes fibers obtained from a combination of
1). polycaprolactone (PCL) / polyethylene oxide (PEO) polymers and an active substance such as aloe vera (AV),
2). polycaprolactone (PCL) polymers , polyethylene oxide (PEO) and active substances such as shea butter (MS), avocado butter (MA), lanolin (L),
3). polyvinyl alcohol (PVA) and aloe vera (AV),
(ii) middle layer, a carrier of active substances, made of polycaprolactone (PCL) and polyethylene oxide (PEO) non-woven fabric,
(iii) an outer layer, pigment carrier layer, comprising a non-woven fabric made of a combination of polymers: polycaprolactone (PCL), polyethylene oxide (PEO) and pigment (PI).

6. A protective patch comprising at least one layer of a polymeric non-woven fabric (i), (ii), (iii), an adhesive layer (A) and a removable cover layer (C) for the adhesive layer (A), **characterized in that** the polymeric layer (i), (ii), (iii) is a mixture of polycaprolactone (PCL) and polyethylene oxide (PEO), and optionally a mixture of polymers mixed with an excipient in one or more of the following combinations:
1) polycaprolactone (PCL) / polyethylene oxide (PEO) / aloe vera (AV), with Aloe Vera in the amount of 5% by weight of the total weight of polymers polycaprolactone (PCL) and polyethylene oxide (PEO),
2) polycaprolactone (PCL) / polyethylene oxide (PEO) / Shea butter (MS) / Avocado butter (MA) / Lanolin (L) with Shea butter (MS) in the amount of 2%, Avocado butter (MA) in the amount of 2% and Lanolin (L) in an amount of 1% by weight based on the total weight of the polymers polycaprolactone (PCL) and polyethylene oxide (PEO),
3) poly(vinyl alcohol) (PVA) / Aloe Vera (AV), Aloe Vera (AV) in the amount of 5% by weight in relation to the weight of PVA
4) polycaprolactone (PCL) / polyethylene oxide (PEO) / pigment (PI) the pigment was added in the amount of 5 to 50% by weight, depending on the planned color scheme in relation to the total weight of polycaprolactone (PCL) and polyethylene oxide (PEO) polymers,
5) polycaprolactone (PCL) / polyethylene oxide (PEO) / hyaluronic acid, with hyaluronic acid in the amount of 5% by weight in relation to the total weight of polycaprolactone (PCL) and polyethylene oxide (PEO) polymers,
6) polycaprolactone (PCL) / polyethylene oxide (PEO) / CBD oil, with CBD oil in the amount of 5% by weight of the total weight of polycaprolactone (PCL) and polyethylene oxide (PEO) polymers,
7) polycaprolactone (PCL) / polyethylene oxide (PEO) / hemp ointment, with hemp ointment in the amount of 5% by weight of the total weight of polycaprolactone (PCL) and polyethylene oxide (PEO) polymers,
wherein the one or more layers together are spot welded, the adhesive (A), preferably silicone, being spot-applied at the welding points (B).

7. The patch according to claim 6, **characterized in that** the weight ratio of polycaprolactone (PCL) and polyethylene oxide (PEO) is 10:1.

8. The patch according to claim 6 or 7, **characterized in that** it comprises:
a) three layers:
(i) an inner layer, which is in direct contact with the body, for this purpose PCL and/or PEO/AV and/or PCL/PEO/MS/MA/L and/or PVA/AV polymer nonwovens were used,
(ii) a middle layer, which is a carrier of active substances, PCL/PEO polymer nonwovens were used for this purpose,
(iii) an outer layer, which is a pigment carrier, being a combination of PCL/PEO/PI fibres, and a silicone adhesive applied to the inner layer;
b) two layers:
(i) an inner layer, which is in direct contact with the body, for this purpose, non-woven fabrics are used that are a combination of PCL/PEO/AV and/or PCL/PEO/MS/MA/L and/or PVA/AV and/or and PCL/PEO,
(ii) a pigment carrier outer layer comprising a combination of PCL/PEO/PI fibres and a silicone adhesive applied to the inner layer
c) one layer (i) with a silicone adhesive applied to it and in direct contact with the body, for this purpose non-woven fabrics made of a combination of fibers were used: PCL/PEO/AV and/or PCL/PEO/MS/MA/L and/or PVA/AV and/or PCL/PEO and PCL/PEO/PI.

9. A method for obtaining a protective material comprising at least one polymer nonwoven layer obtained by electrospinning in an electrostatic field, **characterized in that** it comprises the following steps:
I. preparation of the initial PCL/PEO solution by mixing polycaprolactone (PCL) and polyethylene oxide (PEO) with the predominance of polycaprolactone (PCL) in a mixture of acetone and chloroform with a volume ratio of 70:30 until complete dissolution,
II. transformation of the starting solution obtained in point I into fibers and non-woven fabrics by electrospinning the starting solution in an electrostatic field.
III. spot welding obtained in point II non-woven fabric at temperatures up to 100°C.

10. A method according to claim 9, **characterized in that**:
step I additionally includes the preparation of one or more starting solutions:
1) PCL/PEO/AV by mixing Aloe Vera powder in a mixture of acetone and chloroform with a volume ratio of 70:30 under ultrasound for 1 minute, then adding polycaprolactone (PCL) and polyethylene oxide (PEO) and mixing until completely dissolution of polymeric materials,
2) PCL/PEO/MS/MA/L by mixing Shea Butter, Avocado Butter and Lanolin in a mixture of acetone and chloroform with a volume ratio of 70:30 in a total amount of up to 5% by weight in relation to the total weight of polymers, and then adding polycaprolactone (PCL) and polyethylene oxide (PEO) and mixing until complete dissolution of polymeric materials,
3) PVA/AV by mixing distilled water with poly(vinyl alcohol) (PVA) granulate under the influence of a temperature of about 90 °C until the granulate is completely dissolved, and then cooling the solution to a temperature of about 20 to 25 °C, and then adding Aloe Vera powder to the solution in the amount of 5% and subjected to dissolution for a period of about 12 hours,
4) PCL/PEO/PI by mixing the pigment in a mixture of acetone and chloroform with a volume ratio of 70:30 under ultrasound for 1 minute, and then adding polycaprolactone (PCL) and polyethylene oxide (PEO) and stirring until the materials are completely dissolved polymers, with the pigment added in the amount of 5 to 50% by weight, depending on the color scheme planned to be obtained in relation to the total weight of the polymers.
5) polycaprolactone (PCL) / polyethylene oxide (PEO) / hyaluronic acid, with hyaluronic acid in the amount of 5% by weight in relation to the total weight of polycaprolactone (PCL) and polyethylene oxide (PEO) polymers,
6) polycaprolactone (PCL) / polyethylene oxide (PEO) / CBD oil, with CBD oil in the amount of 5% by weight of the total weight of polycaprolactone (PCL) and polyethylene oxide (PEO) polymers,
7) polycaprolactone (PCL) / polyethylene oxide (PEO) / hemp ointment, with hemp ointment in the amount of 5% by weight of the total weight of polycaprolactone (PCL) and polyethylene oxide (PEO) polymers,
and step II comprises:
transformation of the starting solution of PCL/PEO and one or more starting solutions 1) - 7) obtained in point I into fibers and non-woven fabric, by electrospinning the starting solutions in an electrostatic field using separate nozzles for each starting solution,

11. A method according to claim 10, **characterized in that** a three-layer protective material is obtained by electrospinning a PCL/PEO starting solution and obtaining a first inner layer (i), then electrospinning one or more starting solutions 1) to 7) directly onto the first layer (i) and obtaining the second - middle layer (ii) constituting the carrier of active substances, then electrospinning the starting solution 5) directly onto the second layer (ii) to obtain the third - outer layer (iii).

12. The method according to claim 10, **characterized in that** a three-layer protective material is obtained by electrospinning one or more of the starting solutions 1) to 7) directly and obtaining the first layer - inner (i) and then electrospinning the PCL/PEO starting solution directly onto the first layer and obtaining a second - middle layer (ii), then electrospinning the starting solution 4) directly onto the second layer (ii) to obtain a third - outer layer (iii).

13. A method according to claim 10, **characterized in that** a two-layer protective material is obtained by electrospinning one or more starting solutions 1) to 7) directly and obtaining the first layer - inner and then the starting solution 4) directly on the second layer to obtain the second layer - external (ii).

14. A method according to claim 10, **characterized in that** a single-layer protective material is obtained by simultaneously electrospinning a plurality of starting solutions 1) to 7) and thereby obtaining a first layer (i).

15. Use of material obtained in method according to any of claims 1 - 14 in skin protective patches.
